# EUROPÄISCHE PATENTANMELDUNG

(11) **EP 3 351 164 A1**
(43) Veröffentlichungstag der Anmeldung: **25.07.2018**
(21) Anmeldenummer: 17189693.9
(22) Anmeldetag: 06.09.2017
(51) Int. Cl.: A61B 5/00, A61B 5/11, A61B 5/01, A61B 5/021, A61B 5/024, A61B 5/145

(54) **ARBEITSPLATZANALYSESYSTEM**

(30) Priorität: 18.01.2017 DE 102017100884
(71) Anmelder: aeris GmbH, 85540 Haar (DE)
(72) Erfinder: GLÖCKL, Josef, 85551 Kirchheim (DE)
(74) Vertreter: Peter, Julian

(57) **Zusammenfassung**

Die Erfindung betrifft ein Arbeitsplatzanalysesystem (1) in einem Arbeitsbereich (10) zur Erfassung und Analyse der Anwesenheit einer zu überwachenden Person (P) in mehreren unterschiedlich definierten Zonen (A, B, C) des Arbeitsbereichs (10) und wenigstens der zeitlichen Aufenthaltsdauer (t_{A}, t_{B}, t_{C}) der zu überwachenden Person (P) innerhalb der Zonen (A, B, C), wobei in dem Arbeitsplatzanalysesystem (1) wenigstens eine Erfassungseinrichtung (20) vorgesehen ist, die ausgebildet ist die Anwesenheit und die Aufenthaltsdauer (t_{A}, t_{B}, t_{C}) der zu überwachenden Person (P) in den jeweiligen Zonen (A, B, C) zu detektieren und zu erfassen, und eine Analyseeinrichtung (30) vorgesehen ist, die ausgebildet ist die Anwesenheit und die Aufenthaltsdauer(t_{A}, t_{B}, t_{C}) der zu überwachenden Person (P) in den jeweiligen Zonen (A, B, C) zu analysieren und auszuwerten.

## Beschreibung

Die Erfindung betrifft ein Arbeitsplatzanalysesystem zur Analyse des Bewegungs- und/oder Körperhaltungsprofils einer Person in einem Arbeitsbereich, der in unterschiedlich definierte Zonen aufgeteilt ist.

Die zunehmend an Schreibtischen verbrachte Arbeitszeit erfordert verstärkte ergonomische Maßnahmen zur Verhinderung von Verspannungen, Schmerzen und langfristigen Schäden des Bewegungsapparates, insbesondere bei Personen mit einem hohen Maß an Schreibtischarbeiten.

Solche ergonomischen Maßnahmen dienen neben den genannten gesundheitlichen Verbesserungen auch der Effizienzsteigerung, da durch die Maßnahmen zusätzlich das Denk- und Konzentrationsvermögen gesteigert wird.

Im Stand der Technik sind bereits verschiedene Arbeitsplatzanalysesysteme bekannt, die jedoch darauf abzielen die Eignung zur Vermeidung von Schäden einmalig zu Erfassen und hierfür Optimierungsoptionen zu erkennen. Solche Systeme haben jedoch den Nachteil, dass sie eine Person nicht motivieren für die Gesundheit förderliche Maßnahmen und Möglichkeiten zu nutzen. Ferner sind im Stand der Technik Systeme bekannt, die zwar einen Hinweis zur Verbesserung geben, jedoch keine Möglichkeit zur Erfassung der tatsächlichen Aktivitäten bzw. der tatsächlichen Haltung der Person umfassen.

Der Erfindung liegt deshalb die Aufgabe zugrunde, die vorbenannten Nachteile zu überwinden und ein Arbeitsplatzanalysesystem zur Verfügung zu stellen, das Bewegungsräume, die Körperhaltung und die Zeiten in denen sich eine Person in den Bewegungsräumen bzw. in den Körperhaltung befindet erfasst, analysiert, extrapoliert und mit vorbestimmten oder von der Person vorgegebenen Werten vergleicht. Ferner hat das Arbeitsplatzanalysesystem die Aufgabe die erfassten Daten zu speichern, anzuzeigen und die Person bei dem Erreichen eines vorgegebenen Wertes zu informieren und zu motivieren zumindest die Körperhaltung zu ändern.

Diese Aufgabe wird durch die Merkmalskombination gemäß Patentanspruch 1 gelöst.

Erfindungsgemäß wir ein Arbeitsplatzanalysesystem in einem Arbeitsbereich zur Erfassung und Analyse der Anwesenheit und/oder von Kennwerten einer zu überwachenden Person in mehreren unterschiedlich definierten Zonen des Arbeitsbereichs und wenigstens der zeitlichen Aufenthaltsdauer der zu überwachenden Person innerhalb der Zonen vorgeschlagen. In dem Arbeitsplatzanalysesystem ist wenigstens eine Erfassungseinrichtung, die dazu ausgebildet ist die Anwesenheit und die Aufenthaltsdauer der zu überwachenden Person in den jeweiligen Zonen zu detektieren und zu erfassen, und eine Analyseeinrichtung vorgesehen, die ausgebildet ist die Anwesenheit und die Aufenthaltsdauer der zu überwachenden Person in den jeweiligen Zonen zu analysieren und auszuwerten.

Ferner ist die Erfassungseinrichtung in einer vorteilhaften Ausbildungsvariante dazu ausgebildet, die Körperhaltung, die Körpertemperatur, die Aufmerksamkeit und/oder die Konzentrationsfähigkeit der zu überwachenden Person zu erfassen, wobei die Analyseeinrichtung ferner dazu ausgebildet ist die Körperhaltung, die Körpertemperatur, die Aufmerksamkeit und/oder die Konzentrationsfähigkeit der zu überwachenden Person zu analysieren, zu bewerten, mit Sollwerten zu vergleichen und/oder anzuzeigen. Die Konzentrationsfähigkeit ist beispielsweise dadurch zu Erfassen, dass über die Mimik, den Neigungswinkel des Kopfes und/oder die Intervalle zwischen Blinzeln und die Dauer des Blinzelns die Müdigkeit bzw. die Erschöpfung der zu überwachenden Person ermittelt wird.

Vorteilhaft ist in einer weiteren vorteilhaften Ausbildungsform, dass in einer ersten Zone der unterschiedlich definierten Zonen ein Sitzarbeitsplatz vorgesehen ist und/oder wobei in einer zweiten Zone der unterschiedlich definierten Zonen ein Steharbeitsplatz vorgesehen ist. Der Sitzarbeitsplatz umfasst dabei eine geeignete Arbeitsfläche, wie beispielsweise einen Schreibtisch oder eine Montagestation, sowie einen Sitzplatz. Der Steharbeitsplatz umfasst neben einer hierfür geeigneten Steh-Arbeitsfläche, an der im Stehen gearbeitet werden kann, einen geeigneten Stehplatz. Der Stehplatz kann beispielsweise einen Steh-Hocker und/oder eine aktive Bodenfläche beinhalten, die das stehen bequemer machen/macht und gleichzeitig die Körperhaltung der zu überwachenden Person ergonomisch vorteilhaft beeinflussen.

Vorteilhaft ist ferner, dass bei einer Ausgestaltungsform zumindest an dem Sitzarbeitsplatz ein aktiv-dynamischer Stuhl oder Pendelhocker vorgesehen ist, der die Körperhaltung der zu überwachenden Person während des Sitzens vorteilhaft beeinflusst.

In einer weiteren vorteilhaften Ausgestaltungsform werden von der wenigstens einen Erfassungseinrichtung Kennwerte umfassend die Körpertemperatur und/oder die Herzfrequenz und/oder die Herzfrequenzvariabilität und/oder den Puls und/oder den Blutdruck und/oder den Blutzucker und/oder die Fließgeschwindigkeit des Blutes und/oder die Sauerstoffsättigung des Blutes erfasst.

Eine dritte Zone der unterschiedlich definierten Zonen stellt in einer vorteilhaften Ausgestaltungsvariante einen Bewegungsraum zwischen und/oder neben den Zonen dar oder weist einen Bewegungsraum auf. Der Bewegungsraum bzw. die dritte Zone ist direkt zu der ersten oder/und zweiten Zone angrenzend oder davon beabstandet anordenbar. Beispielsweise kann der Bewegungsraum bzw. die dritte Zone im selben Raum vorhanden sein, in dem sich die erste und/oder zweite Zone befinden, die dritte Zone kann aber auch von dem Raum beabstandet in einem zweiten Raum angeordnet sein. Der Bewegungsraum ist dabei eine Fläche auf der sich die zu überwachende Person bewegen kann. Beispielsweise soll ein Auf- und Abgehen in dem Bewegungsraum ermöglich werden, wozu dieser zumindest ca. 3 m² umfasst. In dem Bewegungsraum sind auch weitere Arbeitsstationen vorsehbar, an denen beispielsweise kurzzeitig in einer geduckten oder gestreckten Position zu arbeiten ist.

Vorteilhaft ist für das Arbeitsplatzanalysesystem, dass die Erfassungseinrichtung in einer Weiterbildungsform zumindest einen Sensor umfasst.

Ferner ist Vorteilhaft, dass in einer weiteren Ausbildungsvariante jeweils ein Sensor zur Detektion und zur Erfassung der zu überwachenden Person oder zur Erfassung von Kennwerten der zu überwachenden Person zumindest in der ersten und der zweiten Zone vorgesehen ist.

Die Detektion und die Erfassung der zu überwachenden Person in der dritten Zone sind in einer vorteilhaften Variante durch den jeweils einen Sensor der ersten und/oder zweiten Zone vorgesehen. Beispielsweise ist die zu überwachende Person in der dritten Zone über die Abwesenheit der zu überwachenden Person in der ersten und/oder der zweiten Zone definier- bzw. erfassbar. Ferner ist der Erfassungsbereich des Sensors in der ersten und/oder zweiten Zone in einen Nahbereich und einen Fernbereich teilbar, wobei definierbar ist, dass der Nahbereich die erste bzw. zweite Zone und der Fernbereich eine andere Zone, beispielsweise die dritte Zone, definiert und überwacht. Durch die Teilung des Erfassungsbereichs eines Sensors in einen Nah- und einen Fernbereich, sind zwei Zonen der unterschiedlich definierten Zonen bzw. die zu überwachende Person in zwei Zonen von einem Sensor überwach- bzw. erfassbar und konkret unterscheidbar.

Der Sensor ist in einer Ausbildungsvariante vorteilhafterweise ein Ultraschall-Echo-Sensor oder ein Infrarot-Array-Sensor.

Die Erfassungseinrichtung umfasst bei einer vorteilhaften Ausbildungsvariante ferner eine von der Person tragbare Sensoreinrichtung. Die Sensoreinrichtung ist beispielsweise in Form einer Kapsel, eines Dongles, eines Armbandes oder einer Armbanduhr ausführbar und erfasst beispielsweise die von der zu überwachenden Person zurückgelegten Schritte, die Herzfrequenz, die Anzahl der überwundenen Treppenstufen und/oder sportliche Aktivitäten und übermittelt diese Daten ständig oder intervallweise, drahtlos oder drahtgebunden an die Analyseeinrichtung um diese Daten dort zu analysieren. Dabei ist unterscheidbar in welcher Zone welche Daten aufgenommen wurden, sodass diese eindeutig den Zonen zuordenbar sind. Die drahtlose Übertragung ist insbesondere über die Bluetooth Low Energy Funktechnik realisierbar. Die Sensoreinrichtung ist des Weiteren auf oder unter der Haut der zu überwachenden Person anordenbar. Durch die von der zu überwachenden Person tragbare Sensoreinrichtung ist die zu überwachende Person ferner für das Erfassungssystem von anderen Personen unterscheidbar, sodass die Erfassung bzw. Überwachung von nicht zu überwachenden Personen unterbindbar ist.

Ferner ist bei einer Weiterbildungsform von Vorteil, dass die Erfassungseinrichtung ein Mittel umfasst, um erfasste Daten elektronisch über eine Datenschnittstelle an die Analyseeinrichtung zu übermitteln. Das Mittel ist beispielsweise eine elektrisch leitfähige Verbindung zwischen der Erfassungseinrichtung und der Analyseeinrichtung, wobei die Datenschnittstelle als Adapter zwischen Sensor und der Analyseeinrichtung oder direkt von der Analyseeinrichtung ausbildbar ist. Die Datenschnittstelle dient dabei, falls nötig, der Umwandlung der Daten wie sie von der Erfassungseinrichtung erzeugt werden in eine Form, in der sie von der Analyseeinrichtung interpretierbar sind. Die Datenschnittstelle ist auch als ein Verteiler bzw. eine Sammelstelle ausbildbar, sodass mehrere Sensoren die von ihnen jeweils erfassten Daten über die Datenschnittstelle an die Analyseeinrichtung weiter geben.

Die Analyseeinrichtung ist bei einer weiteren vorteilhaften Ausgestaltungform ausgebildet, von der Erfassungseinrichtung erfasste Daten zu speichern, in Abhängigkeit zu der Zone in der sie erfasst wurden zu kategorisieren, zu verarbeiten, auszuwerten und/oder zu extrapolieren und insbesondere die erfassten Daten mit gespeicherten Sollwerten zu vergleichen. Beim Erreichen oder Annähern von Werten, die durch die Daten repräsentiert werden, an vorgegebene Werte, werden die Werte oder Daten angezeigt, gespeichert oder auf elektronischen Weg an eine Datensammelstelle übermittelt.

In Abhängigkeit der Zonen sieht eine vorteilhafte Weiterbildungsform vor, zu kategorisieren, ob die zu überwachende Person sitzt, steht, abwesend ist oder sich bewegt. In Abhängigkeit der Zonen ist die Körperhaltung der zu überwachenden Person kategorisierbar. Ist die zu überwachende Person in der ersten Zone wird das z. B. als sitzend, in der zweiten Zone z. B. als stehend und in der dritten Zone z. B. als sich bewegend kategorisiert.

Von Vorteil ist, dass das Arbeitsplatzanalysesystem in einer Ausbildungsvariante eine Anzeigevorrichtung umfasst, die dazu ausgebildet ist, die von der Analyseeinrichtung gespeicherten, kategorisierten, verarbeiteten, ausgewerteten und/oder extrapolierten Daten anzuzeigen, wobei die Analyseeinrichtung vorzugsweise ein Einzelplatzrechner und die Anzeigevorrichtung vorzugsweise ein an den Einzelplatzrechner angeschlossener Bildschirm ist.

Vorteilhaft ist bei einer weiteren Weiterbildungsform, dass ferner eine Eingabevorrichtung vorgesehen ist, um personenbezogene Daten und/oder ein personenspezifisches Soll-Aufenthaltsmuster der Person für die jeweiligen Zonen manuell einzugeben oder zu ändern, welches mittels der Analyseeinrichtung mit den tatsächlich erfassten Daten verglichen werden kann. Beispielsweise ist vorgebbar wie viele Stunden pro Arbeitstag im Stehen, im Liegen oder in anderen Körperhaltungen verbracht werden soll. Weiter sind beispielsweise das Alter, der Name, körperliche oder gesundheitliche Besonderheiten und ein Bild der zu überwachenden Person eingeb- bzw. hinterlegbar, wodurch beispielsweise die Daten, die Bedienung der Analyseeinrichtung, Empfehlungen für vorgebbare Werte, die Anzeige der Daten und vieles mehr individualisierbar ist. Das Soll-Aufenthaltsmuster entspricht durch die Kategorisierung in Abhängigkeit der Zonen einem Soll-Körperhaltungsmuster. Sowohl aus dem Soll-Aufenthaltsmuster als auch aus dem Soll-Körperhaltungsmuster werden Vergleichsdaten und vorgegebene Werte gewonnen, die von der Analyseeinrichtung ver- und bearbeitet werden.

Vorteilhafterweise umfasst eine Ausgestaltungsform eine Warneinrichtung, die dazu ausgebildet ist zumindest einen Wert der von der Erfassungseinrichtung erfassten Daten anzuzeigen oder zu symbolisieren.

Hierbei ist ferner eine Weiterbildungsform vorteilhaft, bei der die Warneinrichtung die Aufenthaltsdauer der zu überwachenden Person in der jeweiligen Zone anzeigt und insbesondere ein Erreichen und/oder eine Abweichungen von dem im System hinterlegten Soll-Aufenthaltsmuster anzeigt.

In einer speziellen, vorteilhaften Ausbildungsvariante ist die Warneinrichtung eine Leuchte bzw. Lampe, die mit steigendem Wert der Aufenthaltsdauer in der jeweiligen Zone heller Leuchtet und bei dem Erreichen eines voreingestellten in dem Soll-Aufenthaltsmuster hinterlegten Grenzwertes blinkt, bis sich die zu überwachende Person von der aktuellen Zone in eine andere Zone bewegt bzw. die Körperhaltung ändert.

Die Erfassungseinrichtung ist in einer vorteilhaften Ausbildungsvariante so ausgebildet, dass sie nicht am Körper getragen ist, sondern stationär verbleibt.

Ferner ist in einer weiteren bevorzugten Ausführungsform die Anzeigevorrichtung, die Eingabevorrichtung und/oder die Analyseeinrichtung ein tragbares Mobilgerät.

Vorteilhafterweise ist die zu überwachende Person in einer Weiterbildungsform durch geeignete Maßnahmen von anderen Personen unterscheidbar. Geeignete Maßnahmen sind beispielsweise Bewegungsverfolgung durch die Erfassungseinrichtung oder die Individualisierung und Erkennung der zu überwachenden Person durch Videosysteme oder Sende- und Empfangseinrichtungen wie einem RFID-Sender-Empfänger-System.

Die vorstehend offenbarten Merkmale sind beliebig kombinierbar soweit dies technisch möglich ist und diese nicht im Widerspruch zueinander stehen. Ferner sind in einer vorteilhaften Weiterbildung Mittel vorgesehen um die überwachte Person in Abhängigkeit des ermittelten Aufenthalts- und/oder Körperhaltungsmusters zum Verlassen der Zone in der sie sich befindet zu motivieren. Solche Mittel können beispielsweise Stromunterbrechung in einer Zone durch einen Stromunterbrecher, visuelle oder akustische Hinweise bzw. Nachrichten in einer Zone oder Ähnliches umfassen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Fig. 1: eine schematische Darstellung eines Arbeitsplatzanalysesystems;
- Fig. 2: eine weitere schematische Darstellung eines Arbeitsplatzanalysesystems;
- Fig. 3: eine schematische Darstellung von Sensoren, einer Warneinrichtung und einer Datenschnittstelle;
- Fig. 4: ein Tagesprofil der Körperhaltung bzw. der Aufenthaltsdauern einer zu überwachenden Person in den Zonen.

Die Figuren sind beispielhaft schematisch. Gleiche Bezugszeichen in den Figuren weisen auf gleiche funktionale und/oder strukturelle Merkmale hin.

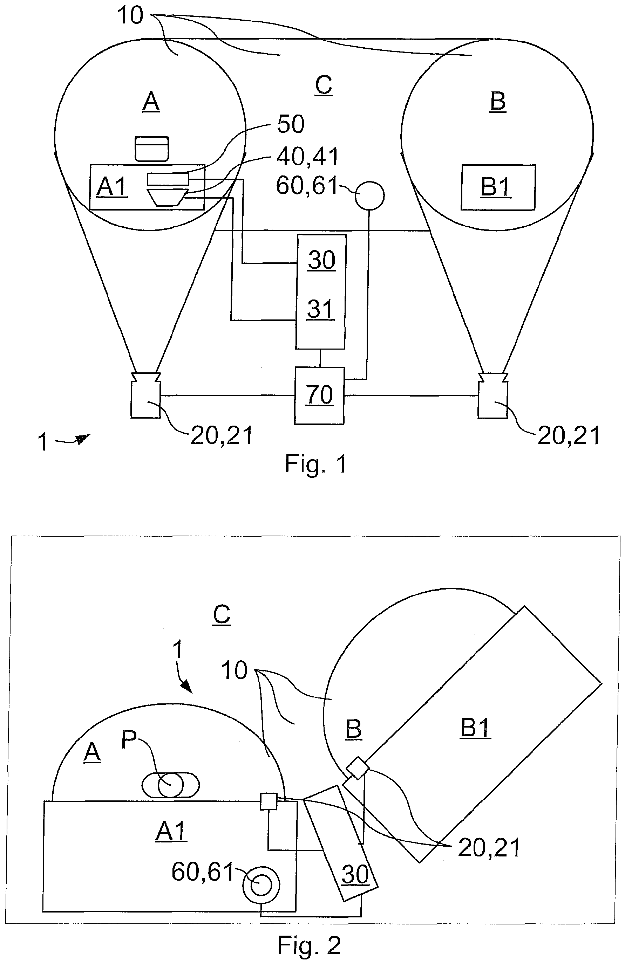

In Figur 1 ist ein Arbeitsplatzanalysesystem 1 dargestellt, wobei der Arbeitsbereich 10 in eine erste Zone A, eine zweite Zone B und eine dritte Zone C unterteilt ist. In der ersten Zone A ist ein Sitzarbeitsplatz A1 angeordnet und in der von der ersten Zone A beabstandeten zweiten Zone B ein Steharbeitsplatz B1. Zwischen der ersten und der zweiten Zone A, B ist die dritte Zone C vorgesehen, die einen Bewegungsraum bildet, in dem eine nicht dargestellte zu überwachende Person sich bewegen kann. In der ersten Zone A, im Bereich des Sitzarbeitsplatzes A1, der von einem Schreibtisch als Arbeitsfläche und einem aktiv-dynamischen Stuhl bestimmt ist, ist die als eine Tastatur ausgebildete Eingabevorrichtung 50 und die als der Bildschirm 41 ausgebildete Anzeigevorrichtung 40 angeordnet. Sowohl die Eingabevorrichtung 50 als auch die Anzeigevorrichtung 40 sind elektronisch mit der Analyseeinrichtung 30 verbunden, die als Einzelplatzrechner 31 ausgebildet ist. Ein Sensor 21 und ein weiterer Sensor 21, die die Erfassungseinrichtung 20 bilden, sind als Ultraschall-Echo-Sensoren ausgebildet. Der Sensor 21 überwacht dabei die Anwesenheit der zu überwachenden Person in der ersten Zone A und der weitere Sensor 21 die Anwesenheit der zu überwachenden Person in der zweiten Zone B. Die Anwesenheit der zu überwachenden Person in der dritten Zone C ist hier durch die Abwesenheit der zu überwachenden Person in der ersten und in der zweiten Zone A, B definiert.

Die Sensoren 21 sind mit einer Datenschnittstelle 70 elektrisch verbunden. Die von den Sensoren erzeugten Daten werden über jeweils eine elektrische Verbindungsleitung zwischen einem Sensor 21 und der Datenschnittstelle 70 an die Datenschnittstelle 70 übermittelt. Die Daten werden in der Datenschnittstelle 70 umgewandelt und gesammelt über eine elektrische Verbindung an die Analyseeinrichtung 30 übermittelt. In der als Einzelplatzrechner 31 ausgebildeten Analyseeinrichtung 30 werden die von der Datenschnittstelle 70 übermittelten Daten ausgewertet, mit hinterlegten Daten verglichen und extrapoliert. Die Ergebnisse bzw. eine graphische Darstellung der Daten und der Auswertungsergebnisse werden über den Bildschirm 41 angezeigt.

Ein Wert, der die aktuelle Aufenthaltsdauer der zu überwachenden Person in der aktuellen Zone, in der sich die zu überwachende Person befindet, repräsentiert, wird beim Erreichen eines ersten vorgegebenen Grenzwerts von der Analyseeinrichtung 30 umgewandelt und über die Datenschnittstelle 70 an die Warneinrichtung 60 übertragen. Die Warneinrichtung 60, die als in der dritten Zone C angeordnete Lampe 61 ausgebildet ist, wird durch den an sie übertragenen Wert ab dem ersten vorgegebenen Grenzwert bis zum Erreichen eines zweiten vorgegebenen Grenzwerts stufenweise heller. Beim Erreichen des zweiten vorgegebenen Grenzwerts, der der Soll-Vorgabe für die maximale Aufenthaltsdauer in der aktuellen Zone an einem Stück entspricht, beginnt die Warneinrichtung 60 bzw. die Lampe 61 zu blinken um zu symbolisieren, dass die zu überwachende Person baldmöglichst die aktuelle Zone verlassen und seine Tätigkeit in einer anderen Zone fortsetzten soll.

In Figur 2 ist ein Arbeitsplatzanalysesystem 1 abgebildet, das ähnlich zu dem in Figur 1 gezeigten Arbeitsplatzanalysesystem 1 ist. Jedoch ist die Datenschnittstelle direkt in die Analyseeinrichtung 30 integriert, sodass die Bestandteile der Erfassungseinrichtung 20 und die Warneinrichtung 60 elektronisch direkt mit der Analyseeinrichtung 30 verbunden sind. Ferner ist die dritte Zone C nicht zwischen der ersten und der zweiten Zone A, B vorgesehen, sondern umschließt diese.

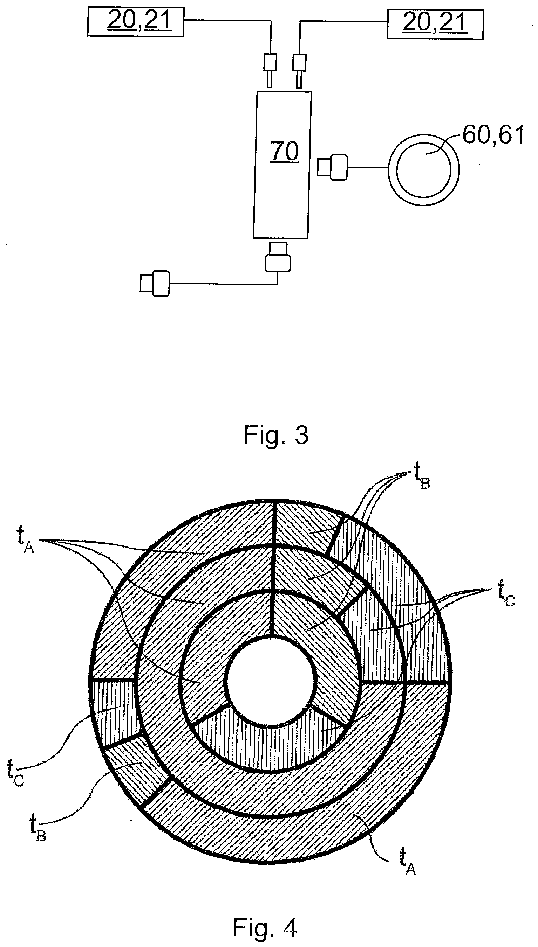

Figur 3 zeigt die Komponenten des Arbeitsplatzanalysesystems 1, die an einem normalen Arbeitsplatz meist noch nicht vorhanden sind. Die als Sensoren 21 ausgebildete Erfassungseinrichtung 20 ist jeweils über ein von den Sensoren 21 ausgehendes Kabel mit der Datenschnittstelle 70 verbindbar. Die Datenschnittstelle 70 weist eine Vielzahl von Steckbuchsen auf, wodurch die Kabel der Sensoren 21 bzw. die Steckkontakte der Kabel mit der Datenschnittstelle 70 verbindbar sind. Die Warneinrichtung 60, die als Lampe 61 ausgebildet ist, ist über ein von ihr ausgehendes USB-Verbindungskabel mit der Datenschnittstelle 70 verbindbar. Die Datenschnittstelle 70 ist über ein USB-Kabel mit der Analyseeinrichtung 30 verbindbar.

Figur 4 zeigt die Verteilung der Anteile von t_{A}, t_{B} und t_{C} an einem Arbeitstag der 8 Stunden umfasst. Der innerste Ring stellt dabei die Sollwerte gesammelt dar, also den Anteil der 8 Stunden die pro Arbeitstag im Sitzen t_{A}, im Stehen t_{B} und in Bewegung t_{C} verbracht werden sollten. Der mittlere Ring stellt die Zeiten t_{A}, t_{B} und t_{C} dar, die tatsächlich von der zu überwachenden Person im Sitzen, Stehen und in Bewegen verbracht werden. Der äußerste Ring stellt ebenfalls die tatsächlichen Zeiten t_{A}, t_{B} und t_{C} dar, wobei diese nicht summiert wurden sondern jeweils die Zeiten darstellen die in der jeweiligen Körperhaltung bzw. Position bzw. Zone am Stück verbracht wurden.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht. Beispielsweise wären mehrere Zonen durch einen einzelnen Sensor gleichzeitig überwachbar oder Zonen durch mehrere Sensoren gleichzeitig überwachbar.

## Patentansprüche

1. Arbeitsplatzanalysesystem (1) in einem Arbeitsbereich (10) zur Erfassung und Analyse der Anwesenheit und/oder von Kennwerten einer zu überwachenden Person (P) in mehreren unterschiedlich definierten Zonen (A, B, C) des Arbeitsbereichs (10) und wenigstens der zeitlichen Aufenthaltsdauer (t_{A}, t_{B}, t_{C}) der zu überwachenden Person (P) innerhalb der Zonen (A, B, C), wobei in dem Arbeitsplatzanalysesystem (1) wenigstens eine Erfassungseinrichtung (20) vorgesehen ist, die ausgebildet ist die Anwesenheit und die Aufenthaltsdauer (t_{A}, t_{B}, t_{C}) der zu überwachenden Person (P) in den jeweiligen Zonen (A, B, C) zu detektieren und zu erfassen, und eine Analyseeinrichtung (30) vorgesehen ist, die ausgebildet ist die Anwesenheit und die Aufenthaltsdauer(t_{A}, t_{B}, t_{C}) der zu überwachenden Person (P) in den jeweiligen Zonen (A, B, C) zu analysieren und auszuwerten.

2. Arbeitsplatzanalysesystem (1) nach Anspruch 1, wobei in einer ersten Zone (A) ein Sitzarbeitsplatz (A1) vorgesehen ist und/oder wobei in einer zweiten Zone (B) ein Steharbeitsplatz (B1) vorgesehen ist.

3. Arbeitsplatzanalysesystem (1) nach Anspruch 2, wobei zumindest an dem Sitzarbeitsplatz (A1) ein aktiv-dynamischer Stuhl oder Pendelhocker vorgesehen ist.

4. Arbeitsplatzanalysesystem (1) nach einem der vorhergehenden Ansprüche, wobei die wenigstens eine Erfassungseinrichtung Kennwerte umfassend die Körpertemperatur und/oder die Herzfrequenz und/oder die Herzfrequenzvariabilität und/oder den Puls und/oder den Blutdruck und/oder den Blutzucker und/oder die Fließgeschwindigkeit des Blutes und/oder die Sauerstoffsättigung des Blutes erfasst.

5. Arbeitsplatzanalysesystem (1) nach einem der vorhergehenden Ansprüche, wobei eine dritte Zone (C) einen Bewegungsraum zwischen oder neben den Zonen (A, B) darstellt oder aufweist.

6. Arbeitsplatzanalysesystem (1) nach einem der vorhergehenden Ansprüche, wobei die Erfassungseinrichtung (20) zumindest einen Sensor (21), insbesondere einen Ultraschall-Echo-Sensor oder ein Infrarot-Array-Sensor, umfasst und wobei jeweils ein Sensor (21) zur Detektion und Erfassung der zu überwachenden Person (P) oder zur Erfassung von Kennwerten der zu überwachenden Person (P) zumindest in der ersten und der zweiten Zone (A, B) vorgesehen ist.

7. Arbeitsplatzanalysesystem (1) nach dem vorhergehenden Anspruch, wobei jeweils die Detektion und Erfassung der zu überwachenden Person (P) in der dritten Zone (C) durch den jeweils einen Sensor (21) der ersten und/oder zweiten Zone (A, B) vorgesehen ist.

8. Arbeitsplatzanalysesystem (1) nach einem der vorhergehenden Ansprüche, wobei die Erfassungseinrichtung (20) ferner eine von der Person (P) tragbare Sensoreinrichtung umfasst.

9. Arbeitsplatzanalysesystem (1) nach einem der vorhergehenden Ansprüche, wobei von der Erfassungseinrichtung (20) ein Mittel umfasst ist, um erfasste Daten elektronisch über eine Datenschnittstelle (70) an die Analyseeinrichtung (30) zu übermitteln.

10. Arbeitsplatzanalysesystem (1) nach einem der vorhergehenden Ansprüche, wobei die Analyseeinrichtung (30) ausgebildet ist, von der Erfassungseinrichtung (20) erfasste Daten zu speichern, in Abhängigkeit zu der Zone (A, B, C) in der sie erfasst wurden zu kategorisieren, zu verarbeiten, auszuwerten und/oder zu extrapolieren und insbesondere die erfassten Daten mit gespeicherten Sollwerten zu vergleichen.

11. Arbeitsplatzanalysesystem (1) nach dem vorhergehenden Anspruch, wobei die Analyseeinrichtung (30) ausgebildet ist, in Abhängigkeit der Zonen (A, B, C) zu kategorisieren, ob die zu überwachende Person (P) sitzt, steht, abwesend ist oder sich bewegt.

12. Arbeitsplatzanalysesystem (1) nach dem vorhergehenden Anspruch, **dadurch gekennzeichnet, dass** es eine Anzeigevorrichtung (40) umfasst, die dazu ausgebildet ist, die von der Analyseeinrichtung (30) gespeicherten, kategorisierten, verarbeiteten, ausgewerteten und/oder extrapolierten Daten anzuzeigen, wobei die Analyseeinrichtung (30) vorzugsweise ein Einzelplatzrechner (31) und die Anzeigevorrichtung (40) vorzugsweise ein an den Einzelplatzrechner (31) angeschlossener Bildschirm (41) ist.

13. Arbeitsplatzanalysesystem (1) nach einem der Ansprüche 10 bis 12, **dadurch gekennzeichnet, dass** ferner eine Eingabevorrichtung (50) vorgesehen ist, um personenbezogene Daten und/oder ein personenspezifisches Soll-Aufenthaltsmuster der Person (P) für die jeweiligen Zonen (A, B, C) manuell einzugeben oder zu ändern, welches mittels der Analyseeinrichtung (30) mit den tatsächlich erfassten Daten verglichen werden kann.

14. Arbeitsplatzanalysesystem (1) nach der Ansprüche 10 bis 13, **dadurch gekennzeichnet, dass** es eine Warneinrichtung (60) umfasst, die dazu ausgebildet ist, zumindest einen Wert der von der Erfassungseinrichtung (20) erfassten Daten anzuzeigen, wobei die Warneinrichtung (60) die Aufenthaltsdauer (t_{A}, t_{B}, t_{C}) der zu überwachenden Person (P) in der jeweiligen Zone (A, B, C) anzeigt und insbesondere ein Erreichen und/oder eine Abweichungen von dem im System hinterlegten Soll-Aufenthaltsmuster anzeigt.

15. Arbeitsplatzanalysesystem (1) nach dem vorhergehenden Anspruch, wobei die Warneinrichtung (60) eine Leuchte (61) ist, die mit steigendem Wert der Aufenthaltsdauer in der jeweiligen Zone (A, B, C) heller leuchtet und bei dem Erreichen eines voreingestellten in dem Soll-Aufenthaltsmuster hinterlegten Grenzwertes blinkt, bis sich die zu überwachende Person (P) von der aktuellen Zone in eine andere Zone bewegt.
